# EUROPEAN PATENT APPLICATION

(11) **EP 1 864 604 A1**
(43) Date of publication of application: **12.12.2007**
(21) Application number: 05721675.6
(22) Date of filing: 30.03.2005
(51) Int. Cl.: A61B 1/00

(54) **WIRELESS DEVICE FOR ACQUIRING INFORMATION ON INSIDE OF SUBJECT AND WIRELESS SYSTEM FOR ACQUIRING INFORMATION ON INSIDE OF SUBJECT**

(71) Applicant: Olympus Corporation, Tokyo 151-0072 (JP)
(72) Inventor: HONDA, Takemitsu Olympus Medical Systems Corp., Tokyo 151-0072 (JP)
(74) Representative: von Hellfeld, Axel
(86) International application number: PCT/JP2005/006177
(87) International publication number: WO 2006/103778

(57) **Abstract**

A system control circuit 26 monitors a drive of a light emitting diode (LED) driving circuit 21 and a charge coupled device (CCD) driving circuit 23, a motor 30 as a function executing unit in a capsule endoscope is driven at a timing for an arbitrarily predetermined period different from a lighting period in which the system control circuit 26 lights a LED 20 and an imaging period in which a CCD 22 captures an intra-subject image, and interference for an image caused by a drive of the motor is prevented. As a result, the function executing unit in the capsule endoscope is driven at an arbitrarily predetermined timing, and image collection and image transmission inside the subject is properly performed.

## Description

### TECHNICAL FIELD

The present invention relates to a wireless in-vivo information acquiring apparatus and a wireless in-vivo information acquiring system for supplying power to each of units in an in-vivo information acquiring apparatus such as a swallowable capsule endoscope to be introduced into a subject, and specifically to a wireless in-vivo information acquiring apparatus and a wireless in-vivo information acquiring system that determine a timing of supplying power to a wireless device.

### BACKGROUND ART

Recently, a capsule endoscope including an imaging function and a radio function has been proposed in the filed of an endoscope. The capsule endoscope is configured to be swallowed by an examinee as a subject for an observation (examination) to travel inside organs (inside of a body cavity) such as a stomach or a small intestine by a peristaltic movement to capture images one by one by using the imaging function, during an observation period until the capsule endoscope is naturally excreted from a body of the examinee.

During the observation period in which the capsule endoscope travels inside the organs, image data captured inside the body cavity by the capsule endoscope is sequentially transmitted to an external device provided outside the subject, through the radio function such as a radio communication, and stored in a memory provided in the external device. By carrying the external device including the radio function and a memory function, the examinee can move without inconvenience during the observation period from when the examinee swallows the capsule endoscope until the capsule endoscope is excreted. After the observation is finished, a doctor or a nurse makes a diagnosis by displaying body cavity images on a display unit such as a display, based on the image data stored in the memory of the external device.

The above type of the capsule endoscope includes such a swallowable type as disclosed in Patent Document 1. Such a capsule endoscope has been proposed that includes a reed switch that switches ON/OFF by an external magnetic field for controlling a drive of the capsule endoscope and is accommodated in a package that includes a permanent magnet for providing the external magnetic field. In other words, the reed switch provided in the capsule endoscope has a configuration to keep OFF state under a circumstance in which a magnetic field having larger strength than a predetermined strength is provided, and to switch to ON state as the strength of the external magnetic field decreases. Accordingly, when accommodated in the package, the capsule endoscope is not activated. By removing the capsule endoscope from the package when the capsule endoscope is swallowed, the capsule endoscope is moved away from the permanent magnet, to be unaffected from the magnetic strength, and starts being activated. With the above configuration, when the capsule endoscope is accommodated in the package, the capsule endoscope is not to be activated, and after the capsule endoscope is removed from the package, the capsule endoscope captures an image by the imaging function and transmits an image signal by the radio function.

Patent Document 1: International application No. 01/35813 pamphlet

### DISCLOSURE OF INVENTION

### PROBLEM TO BE SOLVED BY THE INVENTION

However, the above apparatus includes a capsule endoscope including a power source such as a motor for performing an attitude control or for injecting a medical agent. When the capsule endoscope is removed from the package and inserted into the subject, and if the power source is activated together with the imaging device and the radio device, there is a problem that a peak current gets high and the power stored in the capsule endoscope is more and more wasted.

Further, when the power source is being activated, a noise is generated on a power line for supplying power to each of the function executing units such as the imaging device and the radio device. Therefore, if the power source and the imaging device are activated simultaneously, there is a threat that the imaging device erroneously performs an operation and an image noise appears on image data while the imaging device captures an intra-subject image. As a result, there is such a problem that good intra-subject image data cannot be obtained by the imaging device.

For an imaging rate of the capsule endoscope, there includes an imaging rate in an imaging mode set for capturing two frames (screens) per second by 500 ms (hereinafter, "normal mode"), and an imaging rate in an imaging mode set for capturing 30 frames per second by 33 ms (hereinafter, "high-speed mode"). Each of the imaging rates is switched over based on an instruction by a radio signal from the external device on a request basis. In other words, because the capsule endoscope travels at a high speed through an esophagus among organs inside the subject, it is required to capture an image in the high-speed mode, and because the capsule endoscope travels at a low speed through a small intestine, it is required to capture an image in the normal mode. An instruction for a mode switching is performed by transmitting a radio signal from the external device, based on an operation by a doctor, and the imaging device captures an image of each of the organs inside the subject and acquires image data at an optimal imaging rate.

However, there is a problem that good image data cannot be obtained if the mode switching is performed based on the instruction from the external device while the image is being captured with a specific mode. For example, for capturing an image in the normal mode, a light emitting diode (LED) as an illuminating unit is set to be repeatedly lighted up at 500 ms intervals, while for capturing an image in the high-speed mode, the LED is set to be lighted up without intervals. At this state, if the imaging mode is switched to the high-speed mode while an image is being captured in the normal mode, the LED is to be lighted up without intervals from a time when the imaging mode is switched. An image captured in the above situation becomes a white image in which a contrast of brightness or of a color is lost. If the imaging mode is switched to the normal mode while an image is being captured in the high-speed mode, the LED is repeatedly lighted up at 500 ms intervals. Accordingly, an image is to be captured while the LED is lighted out, and therefore, there is such a problem that dark image data in which the subject cannot be identified is captured and good image data cannot be obtained.

The present invention has been made in view of the above problems and an object of the present invention is to provide a wireless in-vivo information acquiring apparatus and a wireless in-vivo information acquiring system that realize to properly perform image collection and image transmission inside the subject, by activating the function executing units in the capsule endoscope at an arbitrarily predetermined timing.

Still another object of the present invention is to provide a wireless in-vivo information acquiring apparatus and a wireless in-vivo information acquiring system that realize to properly perform the image collection by switching the imaging mode of the capsule endoscope at an arbitrarily predetermined timing.

### MEANS FOR SOLVING PROBLEM

To solve the problems described above and achieve the object, a wireless in-vivo information acquiring apparatus according to the present invention includes an acquiring unit that acquires information on an inside of a subject into which the wireless in-vivo information acquiring apparatus is inserted; a first driving unit that drives the acquiring unit; a radio transmitting unit that transmits the information acquired by the acquiring unit to an outside of the subject wirelessly; a function executing unit that executes a predetermined function; and a second driving unit that drives the function executing unit in a different time period from a time period in which the first driving unit drives the acquiring unit.

The wireless in-vivo information acquiring apparatus according to the invention as set forth in claim 2 further includes a radio receiving unit that receives a radio signal from the outside of the subject, wherein the second driving unit drives the function executing unit in a different time period from a time period in which the acquiring unit is driven based on an instruction signal received wirelessly by the radio receiving unit.

A wireless in-vivo information acquiring apparatus according to the invention as set forth in claim 3 includes an acquiring unit that acquires information on an inside of a subject into which the wireless in-vivo information acquiring apparatus is inserted; a first driving unit that drives the acquiring unit and causes the acquiring unit to acquire information in a different speed mode; a switching instructing unit that instructs a switching of the mode so that a driving period for driving the acquiring unit by the first driving unit and a switching period for switching the mode are to be in time series; and a radio transmitting unit that transmits the information acquired by the acquiring unit to an outside of the subject wirelessly.

The wireless in-vivo information acquiring apparatus according to the invention as set forth in claim 4 further includes a radio receiving unit that receives a radio signal from the outside of the subject, wherein the switching instructing unit instructs the switching of the mode based on an instruction signal received wirelessly by the radio receiving unit, so that a driving period for driving the acquiring unit and a switching period for switching the mode are to be in time series.

In the wireless in-vivo information acquiring apparatus according to the invention as set forth in claim 5, the acquiring unit includes an illuminating unit that outputs an illuminating light for illuminating the inside of the subject and an imaging unit that acquires an image information on the inside of the subject illuminated by the illuminating unit.

In the wireless in-vivo information acquiring apparatus according to the invention as set forth in claim 6, the function executing unit is a drive executing unit that executes a predetermined driving function in the wireless in-vivo information acquiring apparatus.

A wireless in-vivo information acquiring system according to the invention as set forth in claim 7 includes a wireless in-vivo information acquiring apparatus to be inserted into a subject; and a communication device that is provided outside the subject and acquires information acquired by the wireless in-vivo information acquiring apparatus, via a radio communication, the wireless in-vivo information acquiring apparatus includes an acquiring unit that acquires information on an inside of the subject into which the wireless in-vivo information acquiring apparatus is inserted; a first driving unit that drives the acquiring unit; a radio transmitting unit that transmits the information acquired by the acquiring unit to an outside of the subject wirelessly; a function executing unit that executes a predetermined function; and a second driving unit that drives the function executing unit in a different time period from a time period in which the first driving unit drives the acquiring unit, and the communication device includes a radio receiving unit that wirelessly receives information communicated wirelessly.

In the wireless in-vivo information acquiring system according to the invention as set forth in claim 8, the communication device further includes a radio transmitting unit that transmits a predetermined instruction signal wirelessly, the wireless in-vivo information acquiring apparatus further comprises a radio receiving unit that receives a radio signal from the outside of the subject, and the second driving unit drives the function executing unit in a different time period from a time period in which the acquiring unit is driven, based on an instruction signal received wirelessly by the radio receiving unit.

A wireless in-vivo information acquiring system according to the invention as set forth in claim 9 includes a wireless in-vivo information acquiring apparatus to be inserted into a subject; and a communication device that is provided outside the subject and acquires information wirelessly acquired by the wireless in-vivo information acquiring apparatus, the wireless in-vivo information acquiring apparatus includes an acquiring unit that acquires information on an inside of the subject into which the wireless in-vivo information acquiring apparatus is inserted; a first driving unit that drives the acquiring unit and causes the acquiring unit to acquire information in a different speed mode; a switching instructing unit that instructs a switching of the mode so that a driving period for driving the acquiring unit by the first driving unit and a switching period for switching the mode are to be in time series; and a radio transmitting unit that transmits the information acquired by the acquiring unit to an outside of the subject wirelessly, and the communication device includes a radio receiving unit that wirelessly receives information communicated wirelessly.

In the wireless in-vivo information acquiring system according to the invention as set forth in claim 10, the communication device further includes a radio transmitting unit that transmits a predetermined instruction signal wirelessly, the wireless in-vivo information acquiring apparatus further comprises a radio receiving unit that receives a radio signal from the outside the subject, and the switching instructing unit instructs a switching of the mode so that a driving period for driving the acquiring unit and a switching period for switching the mode are to be in time series.

### EFFECT OF THE INVENTION

The wireless in-vivo information acquiring apparatus and the wireless in-vivo information acquiring system according to the present invention activate a drive executing unit (function executing unit) in a period other than a period for activating the imaging unit. Accordingly, image collection and image transmission inside a subject can be properly performed by activating the function executing unit in the capsule endoscope at an arbitrarily predetermined timing.

The wireless in-vivo information acquiring apparatus and the wireless in-vivo information acquiring system according to the present invention implement an imaging period for activating the imaging unit and a switching period for switching the mode of acquiring image information by the imaging unit in time series.
Accordingly, the image collection and the image transmission inside a subject can be properly performed by switching the imaging mode in the capsule endoscope at an arbitrarily predetermined timing.

### BRIEF DESCRIPTION OF DRAWINGS

FIG. 1 is a system schematic view for describing a concept of a wireless in-vivo information acquiring system according to an embodiment of the present invention;
FIG. 2 is a block diagram of an internal configuration of a capsule endoscope shown in FIG. 1 according to a first embodiment of the present invention;
FIG. 3 is a block diagram of an internal configuration of a communication device shown in FIG. 1 according to the first embodiment;
FIG. 4 is a flowchart for describing an operation of the capsule endoscope shown in FIG. 2;
FIG. 5 is a time chart for each of components of the capsule endoscope shown in FIG. 2;
FIG. 6 is a block diagram of a circuit configuration of the capsule endoscope shown in FIG. 1 according to a second embodiment of the present invention; and
FIG. 7 is a time chart for describing an operation of the capsule endoscope shown in FIG. 6.

### EXPLANATIONS OF LETTERS OR NUMERALS

- 1: Subject
- 2: Capsule endoscope
- 3: Communication device
- 4: Display device
- 5: Portable recording medium
- 20: Light emitting diode (LED)
- 21: LED driving circuit
- 22: Charge coupled device (CCD)
- 23: CCD driving circuit
- 24: RF transmitting unit
- 25: Transmitting antenna unit
- 26: System control circuit
- 27: Tank
- 28: Control-signal detecting circuit
- 29: Valve
- 30: Motor
- 31: Transmitting/receiving jacket
- 32: External device
- 33: RF receiving unit
- 34: Image processing unit
- 35: Storage unit
- 36: Oscillator
- 37: Control-signal input unit
- 38: Superposed circuit
- 39: Amplifier circuit
- 40: Power supply unit
- 41: Receiving antenna unit
- 42: Separator circuit
- 43: Power reproducing circuit
- 44: Booster circuit
- 45: Capacitor
- 46: Flip/flop circuit
- A1 to An: Receiving antenna
- B1 to Bm: Transmitting antenna
- VD: Identification number

### BEST MODE(S) FOR CARRYING OUT THE INVENTION

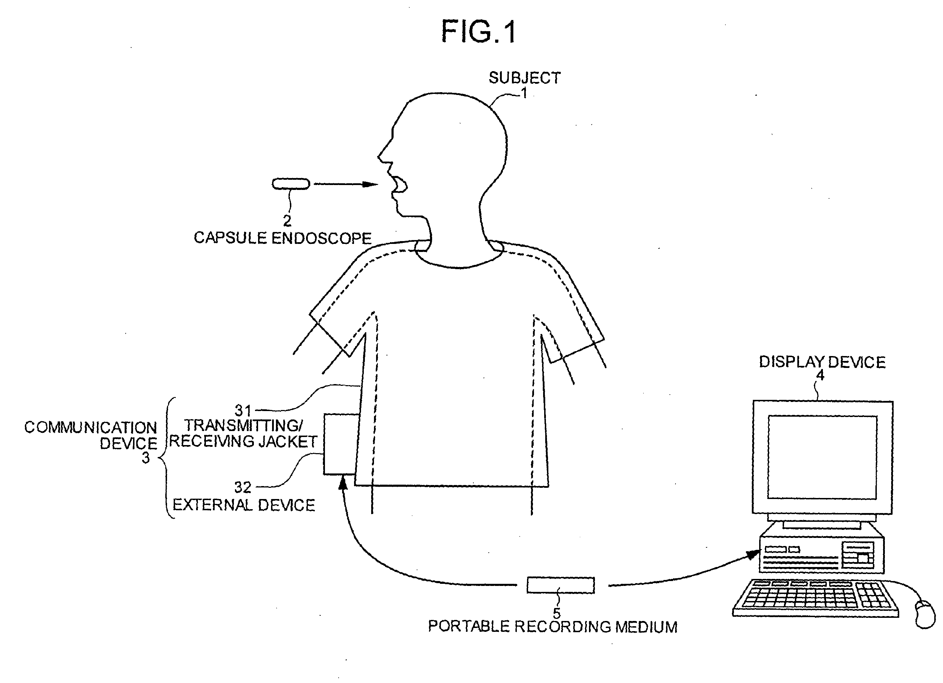

Exemplary embodiments of a wireless in-vivo information acquiring apparatus and a wireless in-vivo information acquiring system according to the present invention are described below in detail with reference to the accompanying drawings FIG. 1 to FIG. 7. In the drawings, same components shown in FIG. 1 are assigned with same reference numerals for a convenience of the description. The present invention is not limited to the below embodiments and various modifications can be made without departing from the spirit or scope of the present invention.

### First embodiment

FIG. 1 is a system schematic view for describing a concept of the wireless in-vivo information acquiring system according to an embodiment of the present invention. In FIG. 1, the capsule endoscope system includes a swallowable capsule endoscope 2 as the wireless in-vivo information acquiring apparatus to be inserted into a body cavity of a subject 1, and a communication device 3 that is an extracorporeal device arranged outside the subject 1 for performing radio communication of various types of information with the capsule endoscope 2. The wireless in-vivo information acquiring system includes a display device 4 that displays an image based on data received by the communication device 3, and a portable recording medium 5 that inputs and outputs data between the communication device 3 and the display device 4.

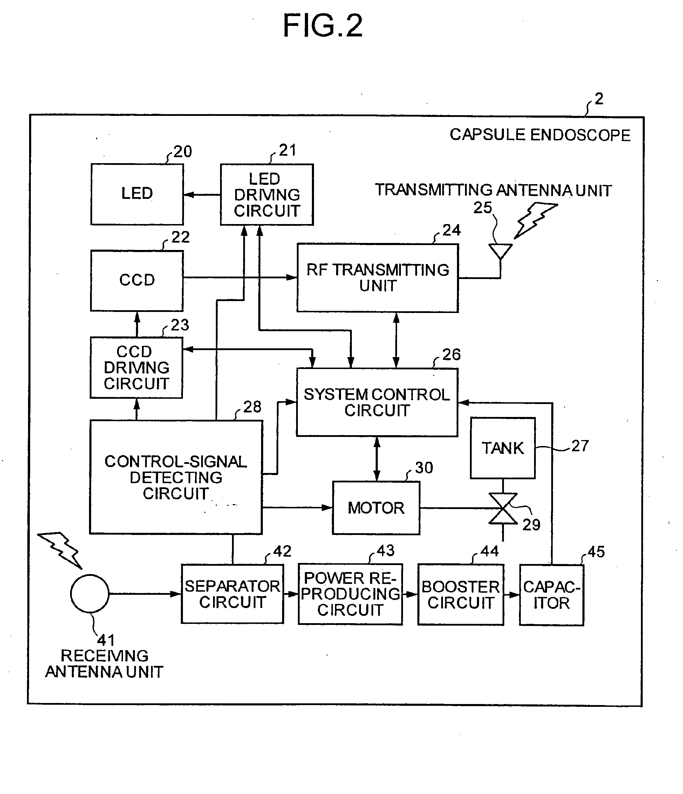

The capsule endoscope 2, as shown in a block diagram of FIG. 2, includes a light emitting diode (LED) 20 as an illuminating unit for illuminating an examined region in the body cavity of the subject 1, a LED driving circuit 21 as a driving unit for controlling a driving state of the LED 20, a charge coupled device (CCD) 22 as an acquiring unit for capturing a body cavity image (in-vivo information) that is a reflected light from a region illuminated by the LED 20, a CCD driving circuit 23 as a first driving unit for controlling a driving state of the CCD 22, a radio frequency (RF) transmitting unit 24 that modulates a captured image signal into an RF signal, and a transmitting antenna unit 25 as a radio transmitting unit for wirelessly transmitting the RF signal output from the RF transmitting unit 24. Further, the capsule endoscope 2 includes a system control circuit 26 that controls an operation of the LED driving circuit 21, the CCD driving circuit 23, and the RF transmitting unit 24, and performs so that the image data of the examined region illuminated by the LED 20 is acquired by using the CCD 22 while the capsule endoscope 2 is inserted in the subject 1. The acquired image data is modulated into the RF signal by the RF transmitting unit 24 and transmitted to the outside of the subject 1 via the transmitting antenna unit 25.

The capsule endoscope 2 includes a tank 27 that stores, for example, medical agent, a valve 29 that injects the medical agent in the tank 27 into the subject 1, and a motor 30 as a function executing unit (drive executing unit) that performs a switching action of the valve 29.
The motor 30 is configured by an alternating-current synchronous motor and an operation of the motor 30 is controlled through a pulse width modulation (PWM) method of the system control circuit 26. In other words, the system control circuit 26 monitors a driving operation for the LED 20 by the LED driving circuit 21, a driving operation for the CCD 22 by the CCD driving circuit 23, and a transmitting operation for the image signal by the RF transmitting unit 24, and performs an operation control of the motor 30 so that operation periods for the above operations are to be different from an operation period of the motor. The motor according to the present invention is not limited to the alternating-current synchronous motor, and can be a direct current motor.

The capsule endoscope 2 further includes a receiving antenna unit 41 as a radio receiving unit that receives a radio signal transmitted from the communication device 3, a separator circuit 42 that separates a feeding signal from a signal received by the receiving antenna unit 41, a power reproducing circuit 43 that reproduces power from the separated feeding signal, a booster circuit 44 that boosts the reproduced power, and a capacitor 45 that stores the boosted power. The capsule endoscope 2 includes a control-signal detecting circuit 28 that detects contents of a control signal from components separated from the feeding signal by the separator circuit 42, and outputs a control signal to the LED driving circuit 21, the CCD driving circuit 23, the system control circuit 26, and the motor 30, if required. The control-signal detecting circuit 28 includes a function of distributing driving power supplied from the capacitor 45 to other components.

With the above mechanisms included, the capsule endoscope 2 receives the radio signal transmitted from the communication device 3 at the receiving antenna unit 41, and separates the feeding signal and the control signal from the received radio signal. The control signal is output, via the control-signal detecting circuit 28, to the LED driving circuit 21, the CCD driving circuit 23, the system control circuit 26, and the motor 30, and used for a drive control of the LED 20, the CCD 22, the RF transmitting unit 24, and the motor 30. On the other hand, the feeding signal is reproduced as the power by the power reproducing circuit 43, and an electric potential of the reproduced power is boosted up to an electric potential of the capacitor 45 by the booster circuit 44 and stored in the capacitor 45. The capacitor 45 has a configuration that enables to supply power to the system control circuit 26 or other components. As described, the capsule endoscope 2 is configured so that the power is supplied by a radio transmission from the communication device 3.

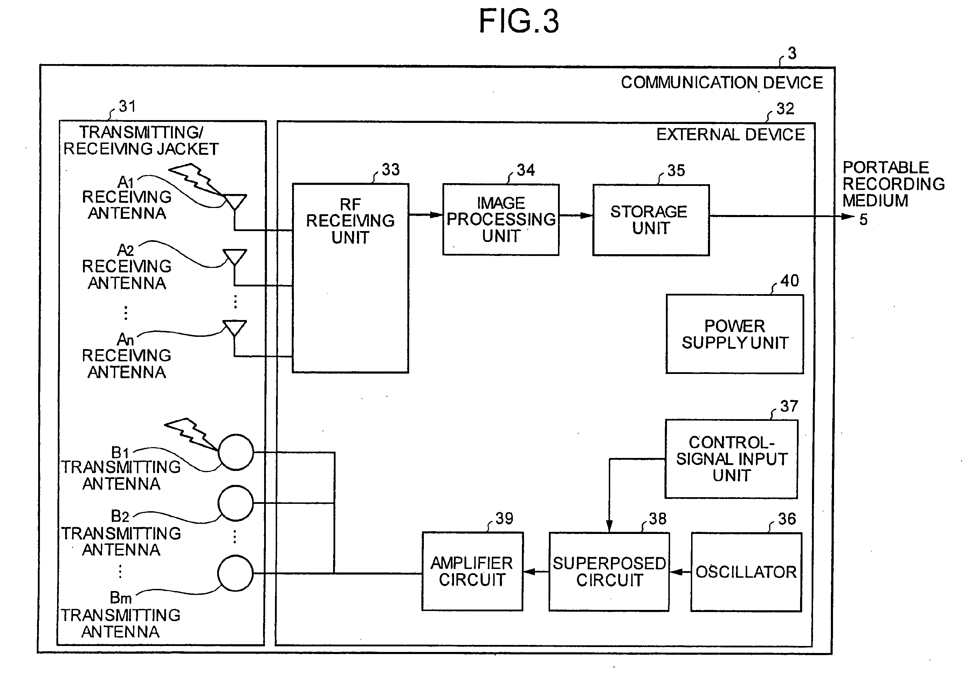

The communication device 3 includes functions of a transmitting device as the radio transmitting unit for transmitting a boot signal to the capsule endoscope 2, and of a receiving device as the radio receiving unit for receiving the image data of the inside of the body cavity transmitted by radio from the capsule endoscope 2. FIG. 3 is a block diagram of an internal configuration of the communication device 3 shown in FIG. 1 according to the first embodiment. In FIG. 3, the communication device 3 includes a transmitting/receiving cloths 31 (i.e., transmitting/receiving jacket) that is to be worn by the subject 1 and includes a plurality of receiving antennas A1 to An and a plurality of transmitting antennas B1 to Bm, and an external device 32 that performs a signal process for the transmitted and received radio signal. At this state, n and m represent arbitral numbers of the antennas set on request basis.

The external device 32 includes an RF receiving unit 33 that performs a predetermined signal process, such as a demodulation, for the radio signal received by the receiving antennas A1 to An and extracts, from the radio signal, the image data acquired by the capsule endoscope, an image processing unit 34 that performs a necessary image process for the extracted image data, and a storage unit 35 for storing the image data for which the image process has been performed, and the external device 32 performs a signal process of the radio signal transmitted by the capsule endoscope 2. According to the present embodiment, the image data is recorded in the portable recording medium 5 via the storage unit 35.

The external device 32 includes a function of generating the radio signal to be transmitted to the capsule endoscope 2, and includes an oscillator 36 that generates the feeding signal and determines an oscillation frequency, a control-signal input unit 37 that generates a control signal for controlling the driving state of the capsule endoscope 2, a superposed circuit 38 that superposes and combines the control signal output from the control-signal input unit 37 to the feeding signal output from the oscillator 36, and an amplifier circuit 39 that amplifies a strength of the combined signal. With the external device 32, the signal combined by the superposed circuit 38 and amplified by the amplifier circuit 39 is output to the transmitting antennas B1 to Bm and transmitted to the capsule endoscope 2. The external device 32 further includes a power supply unit 40 that includes a predetermined electric storage device or an alternating-current (AC) power adaptor, and each of the components of the external device 32 uses a power supplied from the power supply unit 40 as driving energy.

The display device 4 is for displaying a body cavity image captured by the capsule endoscope 2, and has a configuration of, for example, a workstation that performs an image display based on the data obtained by the portable recording medium 5. More specifically, the display device 4 can be configured to directly display an image by a cathode-lay tube (CRT) display or a liquid crystal display, or to output the image to other media such as a printer.

The portable recording medium 5 is connectable to the external device 32 and the display device 4, and includes a configuration that enables to output and record information when the portable recording medium 5 is inserted and connected to the both devices. According to the present embodiment, the portable recording medium 5 is configured so that the portable recording medium 5 is inserted into the external device 32 and records data transmitted from the capsule endoscope 2 while the capsule endoscope 2 travels inside the body cavity of the subject 1. When the capsule endoscope 2 is excreted from the subject 1, that is, when an imaging of the inside of the subject 1 is finished, the portable recording medium 5 is removed from the external device 32 and inserted into the display device 4, and the display device 4 reads out the recorded data onto the display device 4. For example, the portable recording medium 5 is configured by, i.e., a compact flash memory (registered trademark), and a data input and a data output between the external device 32 and the display device 4 can be remotely performed via the portable recording medium 5. As a result, the subject 1 can freely move while the body cavity image is captured, unlike such a case that the external device 32 and the display device 4 are directly connected with a wire.

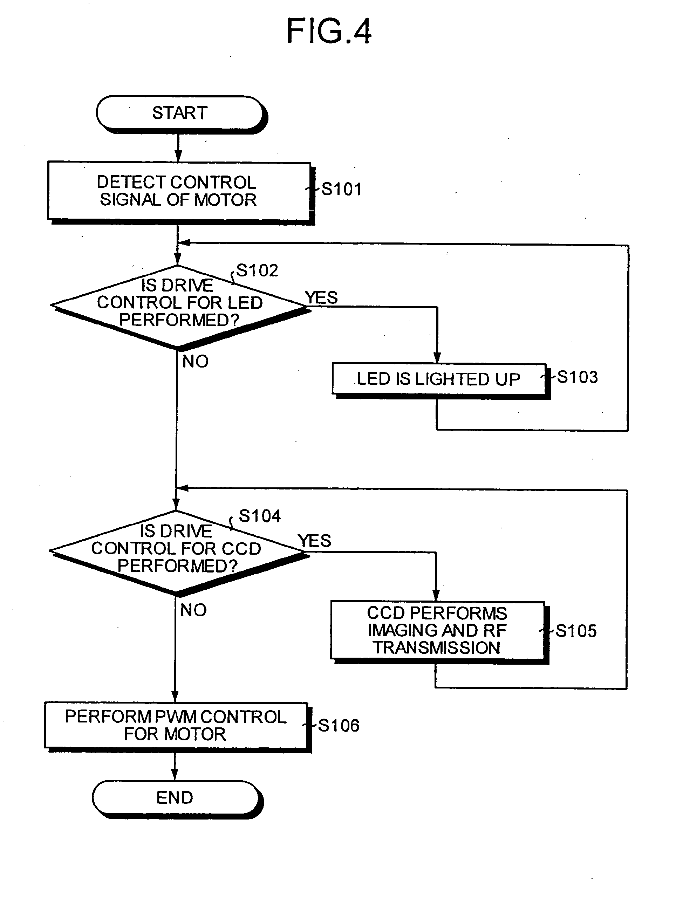

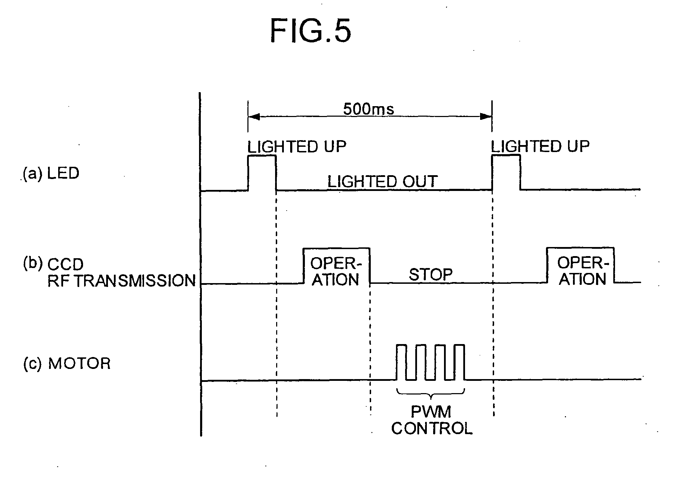

Next, an operation of the capsule endoscope 2 is described with a flowchart shown in FIG. 4 and a time chart shown in FIG. 5. In FIG. 4, the control-signal detecting circuit 28 detects a control signal of the motor 30, which instructs, for example, to turn the motor 30 to the right or turn the motor 30 to the left (step 101), and the system control circuit 26 determines whether the LED driving circuit 21 monitored by the system control circuit 26 has performed a drive control for the LED 20 (step 102).

When the LED driving circuit 21 has performed the drive control for the LED 20, the system control circuit 26 determines that the power has been supplied to the LED 20 and the LED 20 has been lighted up, as shown in FIG. 5(a) (step 103). When the LED driving circuit 21 has not performed the drive control for the LED 20, the system control circuit 26 determines that the LED has been lighted out, and determines whether the CCD driving circuit 23 monitored by the system control circuit 26 has performed a drive control for the CCD 22 (step 104).

When the CCD driving circuit 23 has performed the drive control for the CCD 22, the system control circuit 26 determines that the CCD 22 has captured an image of the inside of the subject 1 and the RF transmitting unit 24 has performed a radio transmission of the image signals, as shown in FIG. 5(b) (step 105). When the CCD driving circuit 23 has not performed the drive control for the CCD 22, the system control circuit 26 determines that an operation of capturing the image of the inside of the subject 1 by the CCD 22 and an operation of performing the radio transmission of the image signals by the RF transmitting unit 24 are stopped, and performs the PWM control for the motor 30 as shown in FIG. 5(c) (step 106), to perform an injection of the medical agent in the tank 27 or to terminate the injection of the medical agent. The control signal of the motor detected by the control-signal detecting circuit 28 is output from the control-signal detecting circuit 28 to the motor 30, and accordingly, a rotation direction of the motor 30 is determined.

As described, according to the present embodiment, the motor is driven in a period other than a lighting period for driving the LED and an imaging period for driving the CCD. Accordingly, a time point for driving the motor can be determined as an arbitrarily predetermined timing. As a result, image collection and image transmission inside the subject can be properly performed.

Further, according to the present embodiment, the LED and the CCD are driven in each of unique periods, which leads to reduce a peak current. Therefore, a waste of the power stored in the capsule endoscope can be decreased, noise in a current line can hardly be generated during the operations of the LED and the CCD, and a false operation of the LED and the CCD can be prevented.

### Second embodiment

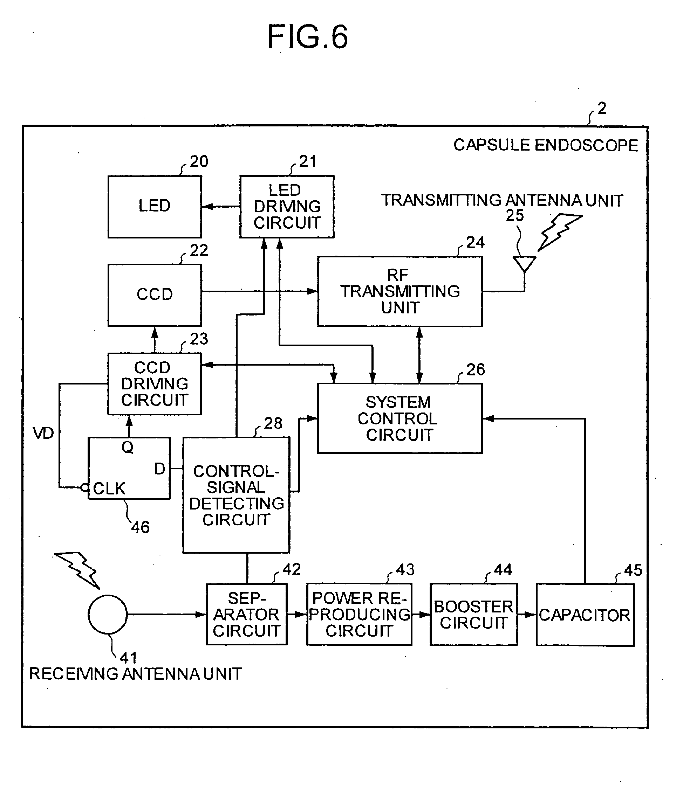

FIG. 6 is a block diagram of a circuit configuration of the capsule endoscope shown in FIG. 1 according to a second embodiment of the present invention. According to the present embodiment, two types of imaging rates including an imaging rate in a normal mode and an imaging rate in a high-speed mode are set for the CCD 22, and the CCD driving circuit 23 is configured to switch a mode based on an instruction by the radio signal from the external device 32.

In FIG. 6, the CCD driving circuit 23 outputs an identifying signal VD for identifying a frame of image data captured at each of the imaging rates, and the identifying signal VD indicates a top of each of the frames. For example, as shown in FIG, 7(b), the identifying signal VD is output from the CCD driving circuit 23 at 500 ms intervals in the normal mode, while the identifying signal VD is output from the CCD driving circuit 23 at 33 ms intervals in the high-speed mode. The CCD 22 outputs the image data of a single screen in frame, at an input timing of the identifying signal VD, to the RF transmitting unit 24.

According to the present embodiment, it is configured to input the control signal (a switching signal that indicates an instruction for switching the mode) to the CCD driving circuit 23 at a timing of the output of the identifying signal VD. More specifically, as shown in FIG. 6, a flip/flop circuit 46 is provided between the CCD driving circuit 23 and the control-signal detecting circuit 28, as a switching instructing unit connected in such a state that an inversion signal of the identifying signal VD from the CCD driving circuit 23 is to be a clock (CLK) and the switching signal from the control-signal detecting circuit 28 is to be an input (D). Further, according to the present embodiment, it is configured to transmit the switching signal from the output (Q) of the flip/flop circuit 46 at an input timing of the inversion signal of the VD to the CCD driving circuit 23.

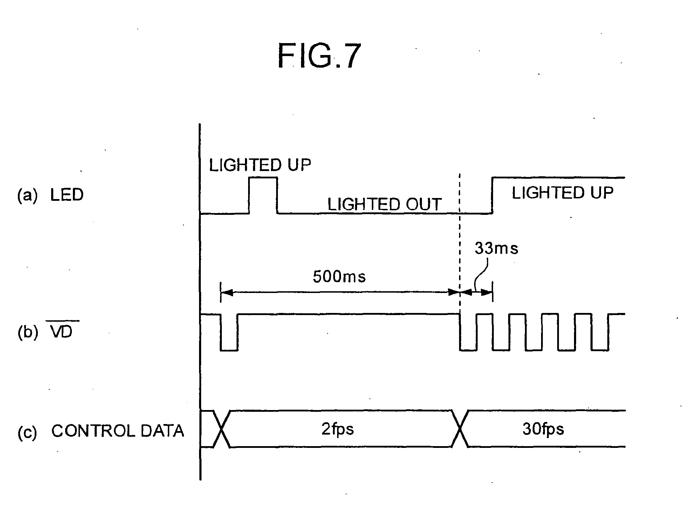

The system control circuit 26 monitors the identifying signal VD output from the CCD driving circuit 2 to control the LED driving circuit 21 to light the LED 20 up at 500 ms intervals, when the identifying signal VD is captured at 500 ms intervals (see, FIG. 7(a)), while to control the LED driving circuit 21 to light the LED 20 up without intervals, when the identifying signal VD is captured at 33 ms intervals (see, FIG. 7(a)). The communication device 3 has the same configuration as shown in FIG. 3, and therefore, the detail description is omitted. The switching signal for switching the mode is generated in the control-signal input unit 37, combined with the feeding signal by the superposed circuit 38, and transmitted as the radio signal from the transmitting antennas B1 to Bm to the capsule endoscope 2.

With the above configuration, when the control-signal detecting circuit 28 detects the switching signal, the control-signal detecting circuit 28 outputs the switching signal as the input (D) of the flip/flop circuit 46. The CCD driving circuit 23 outputs the identifying signal VD at the top of each of the frames of the image data (see, FIG. 7(b)), and when the inversion signal of the VD is captured by the clock (CLK) of the flip/flop circuit 46, the switching signal is latched and temporally transmitted from the output (Q) of the flip/flop circuit 46 to the CCD driving circuit 23.

When the switching signal is input, the CCD driving circuit 23 changes a frequency of the identifying signal VD and outputs the identifying signal VD to the CCD 22, the system control circuit 26, and the flip/flop circuit 46. With the CCD 22, the image data acquired by capturing the body cavity image of the subject 1 in the switched mode is output to the RF transmitting unit 24, as control data of a frame configuration shown in FIG. 7(C).

As described, according to the present embodiment, when the capsule endoscope receives the switching signal for switching the imaging mode from the outside, the switching signal is latched at a timing when the identifying signal VD indicating the frame top is output, and is temporally output to the CCD driving circuit. Accordingly, when an imaging period in a specific mode is finished, the mode is to be switched, and the switching of the imaging mode inside the capsule endoscope is performed at an arbitrarily predetermined timing. As a result, the image collection inside the subject can properly be performed.

According to an embodiment of the present invention, it is acceptable to have such a configuration that the control signal and the switching signal are configured as signals with specific radio frequencies, the signals are transmitted from the external device in the communication device to the capsule endoscope inserted into the body, and the signals with the specific radio frequencies are detected by the control-signal detecting circuit in the capsule endoscope. In this case, it is possible to prevent an erroneous judgment caused by a noise, and to properly perform the image collection and the image transmission for an examination target inside the subject.

### INDUSTRIAL APPLICABILITY

As described above, the body insertable apparatus according to the present invention is suitable for a medical observation apparatus to be inserted into a human body for observing an examined region. More specifically, the body insertable apparatus is suitable for performing a drive of a function executing unit at an arbitrarily predetermined timing to properly perform image collection and image transmission inside a subject.

## Claims

1. A wireless in-vivo information acquiring apparatus comprising:
an acquiring unit that acquires information on an inside of a subject into which the wireless in-vivo information acquiring apparatus is inserted;
a first driving unit that drives the acquiring unit;
a radio transmitting unit that transmits the information acquired by the acquiring unit to an outside of the subject wirelessly;
a function executing unit that executes a predetermined function; and
a second driving unit that drives the function executing unit in a different time period from a time period in which the first driving unit drives the acquiring unit.

2. The wireless in-vivo information acquiring apparatus according to claim 1, further comprising:
a radio receiving unit that receives a radio signal from the outside of the subject, wherein the second driving unit drives the function executing unit in a different time period from a time period in which the acquiring unit is driven based on an instruction signal received wirelessly by the radio receiving unit.

3. A wireless in-vivo information acquiring apparatus comprising:
an acquiring unit that acquires information on an inside of a subject into which the wireless in-vivo information acquiring apparatus is inserted;
a first driving unit that drives the acquiring unit and causes the acquiring unit to acquire information in a different speed mode;
a switching instructing unit that instructs a switching of the mode so that a driving period for driving the acquiring unit by the first driving unit and a switching period for switching the mode are to be in time series; and
a radio transmitting unit that transmits the information acquired by the acquiring unit to an outside of the subject wirelessly.

4. The wireless in-vivo information acquiring apparatus according to claim 3, further comprising:
a radio receiving unit that receives a radio signal from the outside of the subject, wherein the switching instructing unit instructs the switching of the mode based on an instruction signal received wirelessly by the radio receiving unit, so that a driving period for driving the acquiring unit and a switching period for switching the mode are to be in time series.

5. The wireless in-vivo information acquiring apparatus according to any one of claims 1 to 4, wherein the acquiring unit includes an illuminating unit that outputs an illuminating light for illuminating the inside of the subject and an imaging unit that acquires an image information on the inside of the subject illuminated by the illuminating unit.

6. The wireless in-vivo information acquiring apparatus according to claim 1 or 2, wherein the function executing unit is a drive executing unit that executes a predetermined driving function in the wireless in-vivo information acquiring apparatus.

7. A wireless in-vivo information acquiring system that includes: a wireless in-vivo information acquiring apparatus to be inserted into a subject; and a communication device that is provided outside the subject and acquires information acquired by the wireless in-vivo information acquiring apparatus, via a radio communication, the wireless in-vivo information acquiring apparatus comprising:
an acquiring unit that acquires information on an inside of the subject into which the wireless in-vivo information acquiring apparatus is inserted;
a first driving unit that drives the acquiring unit;
a radio transmitting unit that transmits the information acquired by the acquiring unit to an outside of the subject wirelessly;
a function executing unit that executes a predetermined function; and
a second driving unit that drives the function executing unit in a different time period from a time period in which the first driving unit drives the acquiring unit, and the communication device comprising:
a radio receiving unit that wirelessly receives information communicated wirelessly.

8. The wireless in-vivo information acquiring system according to claim 7, wherein the communication device further comprises a radio transmitting unit that transmits a predetermined instruction signal wirelessly,
the wireless in-vivo information acquiring apparatus further comprises a radio receiving unit that receives a radio signal from the outside of the subject, and
the second driving unit drives the function executing unit in a different time period from a time period in which the acquiring unit is driven, based on an instruction signal received wirelessly by the radio receiving unit.

9. A wireless in-vivo information acquiring system that includes: a wireless in-vivo information acquiring apparatus to be inserted into a subject; and a communication device that is provided outside the subject and acquires information wirelessly acquired by the wireless in-vivo information acquiring apparatus, the wireless in-vivo information acquiring apparatus comprising:
an acquiring unit that acquires information on an inside of the subject into which the wireless in-vivo information acquiring apparatus is inserted;
a first driving unit that drives the acquiring unit and causes the acquiring unit to acquire information in a different speed mode;
a switching instructing unit that instructs a switching of the mode so that a driving period for driving the acquiring unit by the first driving unit and a switching period for switching the mode are to be in time series; and
a radio transmitting unit that transmits the information acquired by the acquiring unit to an outside of the subject wirelessly, and the communication device comprising:
a radio receiving unit that wirelessly receives information communicated wirelessly.

10. The wireless in-vivo information acquiring system according to claim 9, wherein the communication device further comprises a radio transmitting unit that transmits a predetermined instruction signal wirelessly,
the wireless in-vivo information acquiring apparatus further comprises a radio receiving unit that receives a radio signal from the outside the subject, and
the switching instructing unit instructs a switching of the mode so that a driving period for driving the acquiring unit and a switching period for switching the mode are to be in time series.
